# EUROPEAN PATENT SPECIFICATION

(11) **EP 2 114 526 B1**
(45) Date of publication and mention of the grant of the patent: **01.08.2012**
(21) Application number: 07835032.9
(22) Date of filing: 28.02.2007
(51) Int. Cl.: A61N 1/37, A61N 1/08, A61N 5/06

(54) **DEVICE FOR DETECTING A MYOCARDIAL INFARCTION AND TREATING THE MYOCARDIAL INFARCTION USING THERAPEUTIC LIGHT**
VORRICHTUNG ZUM NACHWEIS EINES MYOKARDINFARKTS UND ZUR BEHANDLUNG DES MYOKARDINFARKTS MIT THERAPEUTISCHEM LICHT
DISPOSITIF POUR DETECTER UN INFARCTUS DU MYOCARDE ET POUR TRAITER L'INFARCTUS DU MYOCARDE EN UTILISANT UNE LUMIERE THERAPEUTIQUE

(43) Date of publication of application: 11.11.2009
(73) Proprietor: St. Jude Medical AB, 175 84 Järfälla (SE)
(72) Inventor: NORLIN, Anna, S-113 52 Stockholm (SE); HENRIQUEZ, Leda, S-124 32 Bandhagen (SE); STRANDERBERG, Hans, SE-172 65 Sundbyberg (SE); HARSTROM, Eva, SE-165 71 Hasselby (SE); SJOGREN, Mikael, SE-740 83 Fjardhundra (SE); NAESLUND, Anneika, SE-167 64 Bromma (SE); ECKERDAL, Johan, SE-741 92 Knivsta (SE)
(86) International application number: PCT/SE2007/000194
(87) International publication number: WO 2008/105691

(56) References cited:
- EP-A1- 1 690 566
- WO-A1-98/04321
- WO-A1-2008/066423
- WO-A2-03/020103
- US-B1- 6 501 983
- US-B1- 7 181 269

## Description

### TECHNICAL FIELD

The present invention generally relates to cardiac pacing systems and, in particular, to methods and medical devices for detecting and treating myocardial infarctions.

### BACKGROUND OF THE INVENTION

Due to the in general poorer medical status of pacemaker and ICD patients they are subjected to an increased risk of myocardial infarction (MI). The term myocardial infarction refers to the death of myocardial or heart tissue caused by a partial or complete blockage of in one the arteries that supply blood to the heart (coronary arteries), resulting in an interruption in the blood supply to the heart. In the classical acute MI there is a sudden occlusion of a coronary artery due to thrombosis resulting in the death of part of either the right or left ventricular wall. The thrombus occurs due to atheromatous changes in the blood vessel wall.

When heart tissue is deprived of blood-borne oxygen for longer than 30 minutes (called ischemia), it begins to die. Ischemia causes electrical instability within the chambers of the heart, preventing the heart from adequately pump blood throughout the body.

Cardiac repair after MI is a complex process involving diverse inflammatory components, extracellular matrix remodelling and responses of the cardiomyocytes to ischemia. After necrosis of the cardiomyocytes and a long inflammatory phase, the ischemic zone is subsequently replaced by fibrotic tissue. This permanent damage of the heart muscle increases the risk of developing congestive heart failure (CHF).

It is critical to begin treatment of the areas affected by ischemia as soon as possible after the myocardial infarction. Intensive research over the last 20 or more years has demonstrated that prompt treatment can decrease damage from a heart attack and increase the chance for survival. If such therapy is initiated within 1 hour of the inset of symptoms, less irreparable damage may occur.

In light of this, a number of approaches have been made to detect and/or to treat myocardial infarction in implantable medical devices such as pacing devices. For example, in EP 467 695 A2 a method and apparatus for detecting and treating myocardial infarctions in antitachy- arrhythmia and bradycardia pacing devices are disclosed. Electrical activity of the patient's heart is sensed and signalled in order to detect the presence of an MI and a thrombolytic drug is released into the bloodstream upon such detection. Thus, this solution improves the supply of blood at the detection of an MI but, however, it does not treat potential damages of the cardiac tissue caused by the MI.

In EP 1 384 433, by the same applicant, a monitor for early detection of an ischemic heart disease of a patient using intracardiac impedance is shown. According to this solution, the impedance changes due to the increased stiffness of the cardiac tissue caused by the ischemic heart disease are detected. However, EP 1 384 433 is not concerned with the treatment of a detected ischemia.

Furthermore, EP 1 690 566, US 6,604,000 and US 6,256,538 also present implantable medical devices incorporating an ischemia detector responsive to measured intracardiac impedance.

US 2004/0260367 shows a method for treating a detected myocardial infarction of a patient's heart. According to this solution, a light source adapted to generate therapeutic light in the visible to near-infra-red wavelength range using so called low level light therapy ("LLLT") or phototherapy is positioned relative to the patient's heart on the torso of the patient. The therapeutic light penetrates the intervening tissue and the cardiac tissue is irradiated according to a treatment protocol. Thus, the solution according to US 2004/0260367 is impaired with the problem that a detection of the myocardial infarction and a determination of the location of the myocardial infarction are required before the treatment can be initiated. As discussed above, the heart tissue begins to die if it is deprived of blood-borne oxygen for longer than 30 minutes and hence it is critical to begin treatment of the areas affected by ischemia as soon as possible after the myocardial infarction. Therefore, the cardiac tissue may already have been affected with damages, which may be irreparable, when the treatment can be initiated.

WO 2008/066423 describes an implantable medical device and method for improving healing of the trauma following implantation of a medical lead into cardiac tissue by emitting therapeutic light towards areas of damaged cardiac tissue, such as towards the fixation area of fixation means and/or towards the contact area(s) of electrode(s) with the cardiac tissue. The implantable medical device includes a pulse generator adapted to produce cardiac stimulating pulses. Furthermore, the implantable medical device is connectable to at least one lead, which comprises electrodes and at least one fixation means adapted to fixate the lead to cardiac tissue. Light emitting means are arranged to emit therapeutic light towards the fixation area of the fixation means and/or towards at least one contact area of the at least one electrode with the cardiac tissue. In addition, the implantable medical device may comprise a stimulation threshold determining means, which is adapted to determine stimulation threshold values of the at least one contact area of the electrodes. The stimulation threshold may be used as an indicator of the healing process. The therapy provided by the light emitting means may be initiated upon receiving an instruction from a physician.

Thus, there remains a need within the art of a method and medical device that are capable of detecting the occurrence and location of a myocardial infarction and initiating a treatment of the cardiac tissue suffering from the myocardial infarction subsequently to the detection.

### BRIEF DESCRIPTION OF THE INVENTION

Thus, according to an object of the present invention, the occurrence and location of a myocardial infarction is detected and a treatment of the cardiac tissue suffering from the myocardial infarction is initiated.

According to another object of the present invention, the occurrence and location of a myocardial infarction is automatically detected and a treatment of the cardiac tissue suffering from the myocardial infarction is automatically initiated subsequently to the detection.

According to a further object of the present invention, a commencement of a myocardial infarction and the location of the myocardial infarction can be detected at an early stage.

These and other objects of the present invention are achieved by means of an implantable medical device and a medical lead having the features defined in the independent claims. Preferable embodiments of the invention are characterized by the dependent claims.

According to an aspect of the present invention, there is provided an implantable medical device including a pulse generator adapted to produce cardiac stimulating pacing pulses and being connected to at least one medical lead for delivering the pulses to cardiac tissue of a heart of a patient. The implantable medical device comprises myocardial infarction detection means, which myocardial infarction detection means is adapted to detect a myocardial infarction and to identify a location of the myocardial infarction. Further, the implantable medical device comprises a therapy circuit connected to a plurality of light emitting means arranged in the at least one medical lead adapted to emit therapeutic light, and a control circuit connected to the myocardial infarction detection means and to the therapy circuit , the control circuit being adapted to initiate a therapy session via the therapy circuit, wherein one or more of the plurality of light emitting means are selectively activated to emit the therapeutic light towards a detected location of a myocardial infarction at detection of an occurrence of a myocardial infarction.

According to a second aspect of the present invention, there is provided a medical lead connected to an implantable medical device including a pulse generator adapted to produce cardiac stimulating pacing pulses. The medical lead comprises a plurality of electrodes connected to myocardial infarction detection means arranged in the implantable medical device, wherein the myocardial infarction detection means is adapted to detect a myocardial infarction and to identify a location of the myocardial infarction. Further, the medical lead comprises a plurality of light emitting means adapted to emit therapeutic light, the light emitting means being connected to a therapy circuit and to a control circuit arranged in the implantable medical device, wherein the control circuit is adapted to initiate a therapy session via the therapy circuit, wherein one or more of the plurality of light emitting means are selectively activated to emit the therapeutic light towards a detected location of a myocardial infarction at detection of an occurrence of a myocardial infarction.

The invention utilizes the technique photobiomodulation, also called Low Level Laser Therapy (LLLT), Cold Laser Therapy (CLT), Laser Biomodulation, phototherapy or Laser therapy, wherein certain wavelengths of light at certain intensities are delivered for a certain amount of time. More specifically, the present invention is based on the insight of using such therapeutic light to treat cardiac tissue after a myocardial infarction. This is founded upon the findings that photobiomodulation has been proven to be a successful therapy in wound healing see, for example, "Effect of NASA light-emitting diode irradiation on wound healing", H. T. Whelan et al., Journal of Clinical Laser Medicine and Surgery, 19, (2001) p 305. It was also confirmed by Whelan et al. that the cell growth of various cell types in human and rat could be increased by up to 200 % by irradiation of light of certain wavelengths. Furthermore, it has also been shown, for example, in "Low energy laser irradiation reduces formation of scar tissue after myocardial infarction in rats and dogs", U. Oron, et al., Circulation, 103, (2001), p296, that light therapy improves the regeneration of the cardiac cells and decreases the scar tissue formation following a myocardial infarction.

Thus, the present invention provides a number of advantages, for example, an occurrence and location of a myocardial infarction can be detected at an early stage and the treatment of the myocardial infarction can thus be initiated at an early stage. This is of high importance since it has been shown that it is critical to initiate the treatment of the areas of cardiac tissue affected by ischemia as soon as possible after the myocardial infarction. Intensive research over the last 20 or more years has demonstrated that prompt treatment may decrease damage from a heart attack and increase the chance for survival. If a therapy is initiated within 1 hour of the onset of the infarct, less irreparable damage may occur. A further advantage of the present invention is that the regeneration of cardiac cells after a myocardial infarction is improved.

According to an embodiment, the therapy circuit is adapted to activate the light emitting means to emit the therapeutic light according to a treatment protocol, wherein the treatment protocol includes treatment parameters comprising: emitting intervals of the therapeutic light, intensity of the emitted therapeutic light, wavelength of the emitted light, and/or intermittence of the emitted therapeutic light.

In one embodiment, each of the plurality of light emitting means comprises at least one light emitting diode. The light emitting means may, according to embodiments, be arranged in an array along an outer surface of a lead body of respective medical lead.

According to an embodiment of the present invention, the plurality of electrodes is arranged in an array along the outer surface of a lead body of respective medical lead.

In a further embodiment, each of the plurality of light emitting means comprises at least one optical fibre adapted to conduct light emitted from at least one light source arranged in the implantable medical device, wherein the therapy circuit is adapted to selectively activate the at least one light source and/or at least one optical fibre such that light conducted in one or more optical fibres emanates from the one or more optical fibres towards the detected location.

The at least one light source may be a laser source adapted to emit coherent and monochromatic light having a wavelength in the range of 600 nm - 1000 nm. Furthermore, in one embodiment, an intensity of 1-500*mW*/*cm*² and a total dosage of about 1-4*J*/*cm*² are applied. In another embodiment, an intensity of 6-50*mW*/*cm*² and a total dosage of about 1-4*J*/*cm*² may be applied.

According to an embodiment of the present invention, the myocardial infarction detection means comprises an impedance measuring circuit connected to the electrodes arranged in the medical leads. The impedance measuring device is adapted to apply excitation current pulses between respective electrode pairs including at least a first and at least a second electrode and to measure the impedance in the tissues between the at least first and the at least second electrode of the electrode pairs to the excitation current pulses. Further, the myocardial infarction detection means includes a myocardial infarct detector adapted to evaluate the measured impedances by detecting changes in the impedances being consistent with a myocardial infarction and to determine a location of the myocardial infarction using the evaluation. The impedance measuring circuit may measure the impedance between a number of different combinations of electrodes. The impedance measuring circuit may be adapted to periodically initiate impedance measuring sessions according to a myocardial infarction monitoring protocol, wherein the impedance between different pairs of electrodes is measured according to a predetermined sequence (e.g. one pair after another during consecutive cardiac cycles or all pairs simultaneously during a number of consecutive cardiac cycles) to be able to detect and locate a myocardial infarction. That is, during each impedance measuring session, a number of impedance measurments from the different electrode pairs are obtained. Consequently, it is possible to continuously monitor the cardiac tissue to enable a reliable detection of the occurrence and location of a myocardial infarction.

According to embodiments of the present invention, the myocardial infarct detector is adapted to compare measured impedances with a stored reference impedance template to detect an occurrence of a myocardial infarction and a location of the myocardial infarction from the result of the comparison. The template may alternatively be obtained or created by the myocardial infarction detection means during a period when no changes of the monitored signals, e.g. impedance or electrical activity of the cardiac tissue, are of a sufficient magnitude to indicate the possibility of the commencement of a condition such as a myocardial infarction. Such a template may also be updated periodically by performing new measurments of the impedance and/or the electrical activity.

In one example, impedance value ratios for a cardiac cycle is determined by determining a maximum impedance and a minimum impedance, respectively, measured by the impedance measuring circuit during a cardiac cycle. Further, an impedance value ratio being below a predetermined impedance value ratio threshold is determined to be consistent with a myocardial infarction; and the impedance value ratio being smallest of the impedance value ratios being below the predetermined impedance value ratio threshold is determined to indicate the location of the myocardial infarction.

Alternatively, or as a complement to the impedance value ratio determination, the myocardial infarct detector may be adapted to calculate a respective maximum time derivative of the measured impedance , to determine a maximum impedance time derivative being below a predetermined impedance time derivative threshold to be consistent with a myocardial infarction and to determine the maximum impedance time derivative being lowest of the maximum impedance time derivatives being below the predetermined impedance time derivative threshold to indicate the location of the myocardial infarction.

In yet another embodiment of the present invention, the myocardial infarction detection means comprises an intracardiac electrogram measuring circuit connected to the electrodes of respective medical lead and which measuring circuit is adapted to measure intracardiac electrograms using one or more electrodes of the medical leads. Furthermore, the myocardial infarction detection means includes a myocardial infarct detector adapted to evaluate the intracardiac electrograms to detect changes being consistent with a myocardial infarction and to determine a location of the myocardial infarction using the evaluation. A reference template, which template may be a stored reference impedance template, may be used in this evaluation. The template may alternatively be obtained or created by the myocardial infarction detection means during a period when no changes of the monitored signals, e.g. the electrical activity of the cardiac tissue, are of a sufficient magnitude to indicate the possibility of the commencement of a condition such as a myocardial infarction. Such a template may also be updated periodically by performing new measurments of the electrical activity. Consequently, it is possible to continuously monitor the cardiac tissue to enable a reliable detection of an occurrence and location of a myocardial infarction.

Furthermore, according to embodiments of the present invention, a combination of impedance measurements and intracardiac electrograms is used to detect an occurrence and location of a myocardial infarction. Thereby, it is possible to obtain a more reliable detection of the myocardial infarction and the location of the myocardial infarction.

According to embodiments of the present invention, signals being indicative of the healing process of the myocardial infarction is monitored, continuously or periodically, during the therapy session to determine whether the therapy has been successful and should be ended or whether the therapy parameters, i.e. the parameter of the treatment protocol, should be adjusted in order to make the treatment more potent during a certain phase of the healing process or to make the treatment less potent. A more potent treatment may be a higher degree of intensity of light or a constant intensity of light but with a changed intermittence, i.e. longer periods of light delivery or a more frequent light delivery with a constant period of light delivery. A less potent treatment may instead be a lower degree of intensity of light or a constant intensity of light but with a changed intermittence, i.e. shorter periods of light delivery or a less frequent light delivery with a constant period of light delivery.

According to one embodiment of the present invention, the myocardial infarct detector is, after an initiation of a therapy session, adapted to monitor impedances obtained by at least an electrode pair indicating the location of the myocardial infarction to determine whether the impedances indicate that the therapy session should be terminated and/or the treatment parameters should be adjusted or maintained.

Furthermore, the myocardial infarct detector may be adapted to determine impedance value ratios for successive cardiac cycles and to determine that the therapy session should be terminated if a predetermined number of the impedance value ratios are found to be above the impedance value ratio threshold.

In another embodiment, the myocardial infarct detector may be adapted to calculate maximum time derivatives of the measured impedance for successive cardiac cycles and to determine that the therapy session should be terminated if a predetermined number of the maximum impedance time derivatives are found to be above a predetermined impedance time derivative threshold.

According to further embodiments, the myocardial infarct detector is adapted to monitor intracardiac electrograms obtained by at least an electrode pair indicating the location of the myocardial infarction to determine whether the intracardiac electrograms indicate that the therapy session should be terminated and/or the treatment parameters should be adjusted or maintained.

In a certain embodiment, the myocardial infarct detector is adapted to determine ST segments for successive cardiac cycles and determine that the therapy session should be terminated if a predetermined number of the ST segment elevations are found to be below a predetermined ST segment elevation threshold.

Moreover, according to other embodiments of the present invention, a combination of impedance measurements and intracardiac electrograms is used to determine whether the therapy should be terminated or whether the therapy parameters should be adjusted or maintained. For example, both ST segment elevations and maximum impedance time derivatives may be used to evaluate the therapy. Thereby, it is possible to obtain a more reliable judgement of the healing process and the therapy.

According to an embodiment of the present invention, the implantable medical device is provided with means for a power transmission via inductive coupling in order to provide the implantable medical device with additional energy for a healing process. A receiver coil with a rectifier is arranged in the implantable medical device. An external sending coil is arranged to emit AC-fields in frequencies of a few kHz to about 500 kHz. This additional energy may be supplied directly to the light emitting means, for example, the diodes or may be used to charge a re-chargeable battery of the implantable medical device.

In a further embodiment, a warning system is arranged in the implantable medical device adapted to notify the patient (e.g. by means of a beep signal or a created vibration) and/or a care institution such a hospital. For example, the hospital can be notified via message transmitted via an RF (Radio Frequency) unit of the implantable medical device and telecommunication system containing, inter alia, information related to the patient and a detected myocardial infarction stored in the implantable medical device. A decision at the hospital how to proceed with the treatment of the infarct can be based on the transmitted information collected by the sensors of the implantable medical device. For example, medical personnel is able to tune the light therapy by programming the device and the device can be provided with additional power or energy can be supplied from an external power source shortly after the onset of the infarct. The patient is also able to contact medical personnel via a home monitoring equipment installed at his/hers home at notification of a detection of an infarct.

In one embodiment of the present invention, the light emitting means are activated such that therapeutic light is emitted according to a treatment protocol including treatment parameters comprising one, more or all of: emitting intervals of the therapeutic light, intensity of the emitted therapeutic light, wavelength of the emitted light, intermittence of the emitted therapeutic light, or treatment periods. The protocol may thus comprise a predetermined treatment scheme. In an alternative embodiment, the treatment is varied in dependence of one or more treatment response parameters.

In embodiments of the present invention, the light emitting means are adapted to emit light having a wavelength in the range of 600 nm - 1000 nm. Furthermore, an intensity of 1-500*mW*/*cm*² and a total dosage of about 1-4*J*/*cm*² may be used.

The features that characterize the invention, both as to organization and to method of operation, together with further objects and advantages thereof, will be better understood from the following description used in conjunction with the accompanying drawings. It is to be expressly understood that the drawings is for the purpose of illustration and description and is not intended as a definition of the limits of the invention. These and other objects attained, and advantages offered, by the present invention will become more fully apparent as the description that now follows is read in conjunction with the accompanying drawings.

### BRIEF DESCRIPTION OF THE DRAWINGS

In the following detailed description, reference will be made to the accompanying drawings, of which:
Fig. 1 schematically shows an embodiment of a pacemaker system in which an implantable medical device in accordance with the present invention may be implemented;
Fig. 2a schematically illustrates an embodiment of the implantable medical device according to the present invention;
Fig. 2b schematically illustrates another embodiment of the implantable medical device according to the present invention;
Fig. 3a schematically illustrates an embodiment of the myocardial infarction detection means in accordance with the present invention;
Fig. 3b schematically illustrates another embodiment of the myocardial infarction detection means in accordance with the present invention;
Fig. 4 schematically illustrates an embodiment of a medical lead in accordance with the present invention;
Fig. 5 is high-level flow chart of an embodiment of the method for treating a myocardial infarction with therapeutic light using an implantable medical device according to the present invention.

### DETAILED DESCRIPTION OF THE INVENTION

In the following, the present invention will be discussed in the context of medical systems comprising at least an implantable pacemaker, and medical leads such as an atrial lead and a ventricular lead.

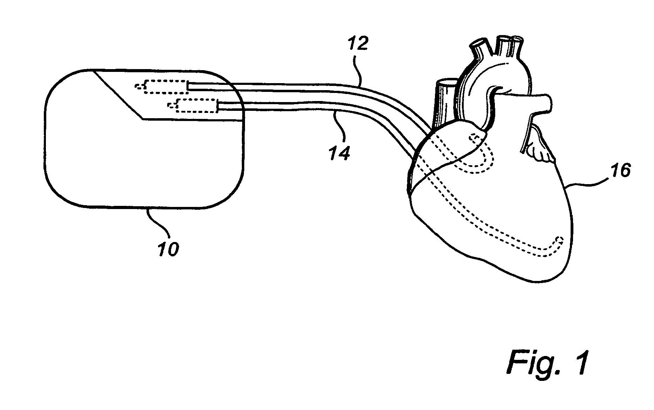

With reference first to Fig. 1, a pacemaker system comprising an implantable pacemaker 10 connectable to an atrial lead 12 and a ventricular lead 14 including electrodes for providing therapy to a heart 16 of a patient. The leads 12, 14 are implanted into the heart 16 via veins and are fixated at the cardiac tissue by means of, for example, helical screws.

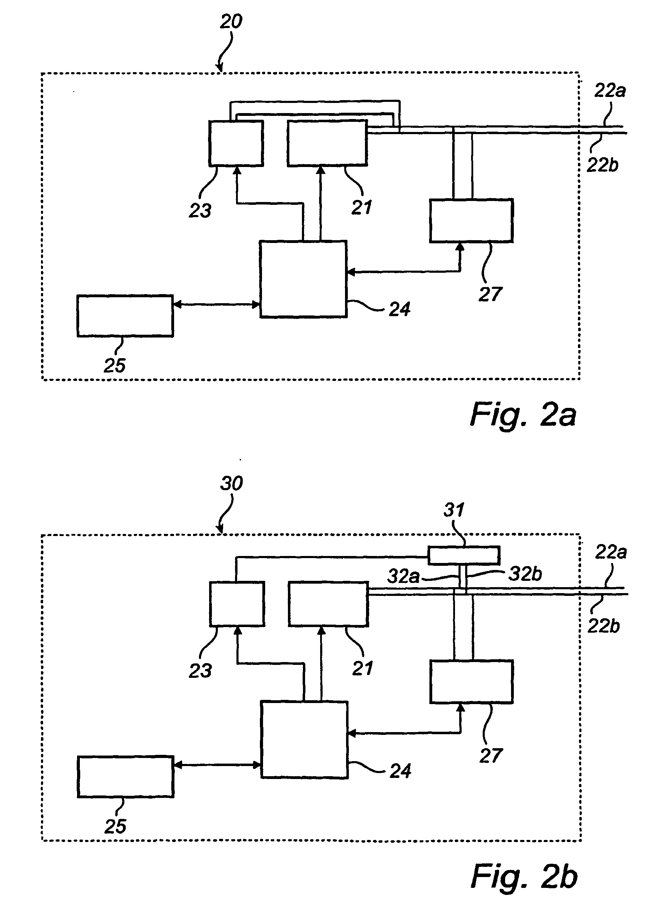

Turning now to Figs. 2a, an embodiment of an implantable medical device, e.g. a pacemaker or an ICD, according to the present invention will be discussed. The implantable medical device 20 comprises a housing (not shown) being hermetically sealed and biologically inert. Normally, the housing is conductive and may, thus, serve as an electrode. The pacemaker 20 is connectable to one or more pacemaker leads, where only two are shown in Figs. 2a and 2b; namely a ventricular lead 22a implanted in the right ventricle of the heart (not shown) and one atrial lead 22b implanted in the right atrium of the heart (not shown).

The leads 22a and 22b can be electrically coupled to the pacemaker 20 in a conventional manner. The leads 22a, 22b comprises one or more electrodes, such as a tip electrode or ring electrodes, arranged to, inter alia, measure the impedance or transmit pacing pulses for causing depolarization of cardiac tissue adjacent to the electrode (-s) generated by a pace pulse generator 21 under influence of a controller or controlling circuit 24 including a microprocessor. The controller 24 controls, inter alia, pace pulse parameters such as output voltage and pulse duration.

Moreover, a storage means 25 is connected to the controller 24, which storage means 25 may include a random access memory (RAM) and/or a non-volatile memory such as a read-only memory (ROM). Storage means 25 is connected to the controller 24. Detected signals from the patient's heart are processed in an input circuit (not shown) and are forwarded to the controller 24 for use in logic timing determination in known manner.

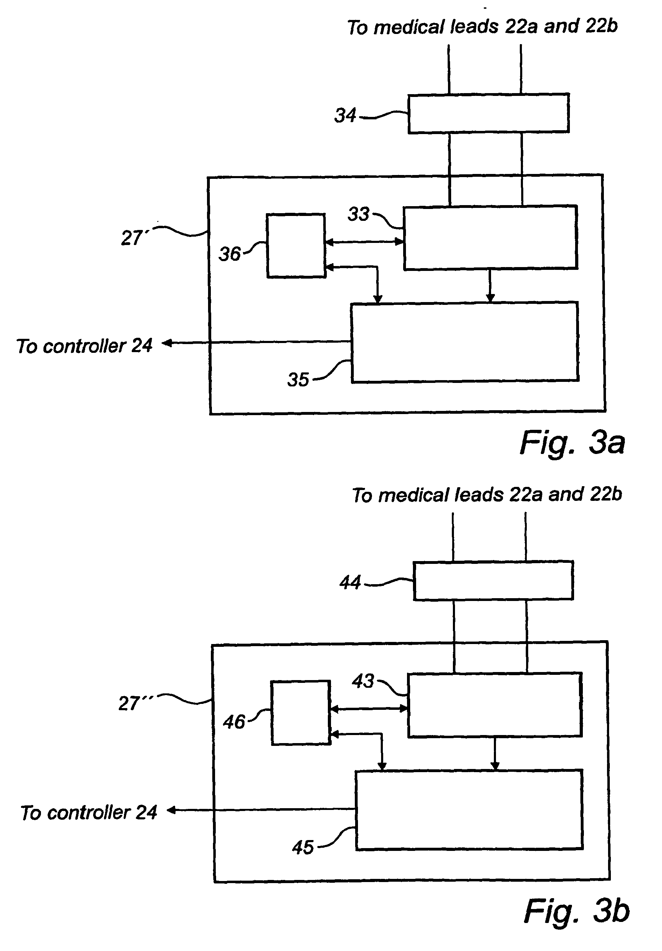

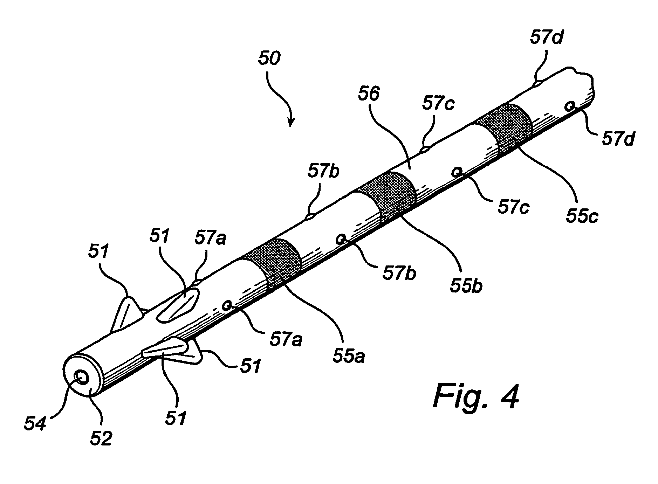

Furthermore, the implantable medical device 20 comprises a myocardial infarction detection means 27, which will be described below in more detail with reference to Figs. 3a and 3b. The myocardial infarction detection means 27 is arranged to process detected signals from the patient's heart to detect whether a myocardial infarction has occurred and may also, if such an infarction is detected, determine or identify a location of the detected myocardial infarction within the heart. Information from the myocardial infarction detection means 27 such as detection of a myocardial infarction and the location may be forwarded to the controller 24. The myocardial infarction detection means 27 is connected to the electrodes arranged in the medical leads 22a, 22b, for example, a number of ring electrodes arranged along the medical leads and tip electrodes, see Fig. 4.

In this embodiment, a plurality of light emitting means (see Fig. 4) are incorporated in one or all of the leads 22a, 22b and connected to the controller 24. In one embodiment, the light emitting means in form of light emitting diodes are arranged at a periphery of the tube-shaped leads 22a and 22b in an array along a longitudinal direction of the leads. The light emitting diodes are adapted to emit monochromatic light having a wavelength of 600 - 1000 nm. The light emitting diodes are connected to a therapy circuit 23, which is adapted to, under control of the controller 27, selectively activate one or more of the diodes. At a detection of a myocardial infarction and at determination of a location within the heart of such an infarction, the myocardial infarction detection means 27 may forward this information to the controller 24, which, in turn, may activate selected diodes via the therapy circuit 23 according to a treatment protocol. One or more of the diodes may be selected, based on the determination of the location of the myocardial infarction, and activated to emit therapeutic light towards the detected myocardial infarction. The treatment protocol may include predetermined or adjustable treatment parameters such as emitting intervals of the therapeutic light, intensity of the emitted therapeutic light, wavelength of the emitted light, and intermittence of the emitted therapeutic light.

The implantable medical device 20 is powered by a battery (not shown), which supplies electrical power to all electrical active components of the implantable medical device 20 including the light emitting means arranged in the medical leads 22a and 22b and the myocardial infarction detection means 27. The implantable medical device 20 may also be provided with means for power transmission via inductive coupling in order to provide the implantable medical device 20 with additional energy for a healing process. A receiver coil (not show) with a rectifier is arranged in the implantable medical device 20. An external sending coil is arranged to emit AC-fields in frequencies of a few kHz to about 500 kHz. This additional energy may be supplied directly to the light emitting means, for example, the diodes or may be used to charge a re-chargeable battery of the implantable medical device 20.

The implantable medical device 20 further comprises a communication unit (not shown), for example, an RF telemetry circuitry for providing RF communications. Thereby, for example, data contained in the storage means 25 can be transferred to an external programmer device (not shown) via the communication unit and a programmer interface (not shown) for use in, for example, analyzing system conditions, patient information, etc.

Moreover, the implantable medical device 20 may further comprise a notifying device (not shown) adapted to, at detection of an occurrence of a myocardial infarction, notify said patient of the event that a myocardial infarct has been detected and/or that therapy has been initiated. In one embodiment, the notifying device is a vibration unit adapted to vibrate in the event that a myocardial infarct has been detected and/or that therapy for treating such an infarct has been initiated and thereby notify the patient.

Referring to Fig. 2b, a further embodiment of the implantable medical device according to the present invention will be discussed. Like parts in the implantable medical device shown in Fig. 2a and Fig. 2b will be denoted with the same reference numerals and descriptions thereof will be omitted since they have been described above with reference to Fig. 2a. A light source 31 is arranged in the implantable medical device 30, for example, a laser adapted to emit monochromatic light having a wavelength of 600 - 1000 nm. The light source 31 is connected to a plurality of optical fibres 32a arranged in the ventricular lead 22a and a plurality of optical fibres 32b arranged in the atrial lead 22b. The optical fibres 32a, 32b are arranged to conduct light emitted by the light source 31 such that the conducted therapeutic light emanates from the optical fibres 32a, 32b towards the cardiac tissue. The optical fibres 32a, 32b are arranged such that light can be applied cardiac tissue along the periphery of the medical leads 22a, 22b. That is, distal ends of respective optical fibres 32a, 32b are arranged in arrays along the outer periphery of the medical leads 22a, 22b see Fig. 4. Furthermore, the light source 31 comprises a selector circuit adapted to, under influence of the controller 27, select one or more of the optical fibres 32a, 32b to conduct light during a therapy session such that therapeutic light can be applied to an identified location of a myocardial infarction.

Referring now to Figs. 3a and 3b, embodiments of the myocardial infarction detection means will be discussed in more detail. With reference first to Fig. 3a, an embodiment of the myocardial infarction detection means adapted to determine an occurrence of a myocardial infarction and to determine the location of the myocardial infarction using measured impedances, for example, transcardiac impedances will be described. The myocardial infarction detection means 27' comprises an impedance measuring circuit 33 connected to the electrodes incorporated in the medical leads 22a and/or 22b, which will be described in more detail below with reference to Fig. 4. In one embodiment, each medical lead comprises a plurality of ring electrodes arranged along the respective lead and a tip electrode and the impedance measuring circuit 33 may be connected to the electrodes via a switching device 34. The switching device 34 may be arranged in the implantable medical device 20 and are adapted to switch an applied current to a selected electrode(-s) of the medical lead (-s) 22a, 22b. A person skilled within the art may design such a switching device based on the switching device described in U.S. 5,423,873. Hence, the impedance measuring circuit 33 may, for example, measure the impedance between a first ring electrode of the first medical lead 22a and the housing the implantable medical device, a first ring electrode of the first medical lead 22a and a second ring electrode of the first medical lead 22a, a first ring electrode of the first medical lead 22a and a first ring electrode of the second medical lead 22b, and first ring electrode of the second medical lead 22b and a second ring electrode of the second medical lead 22b. The impedance measuring circuit 33 is adapted to apply excitation current pulses between respective electrode pair including at least a first and at least a second electrode, as mentioned above, to measure the impedance in the tissues between the at least first and the at least second electrode of the respective electrode pairs to the excitation current pulses. The impedance measuring circuit 33 is adapted to periodically initiate impedance measuring sessions according to a myocardial infarction monitoring protocol, wherein the impedance between different pairs of electrodes is measured according to a predetermined sequence (e.g. one pair after another during consecutive cardiac cycles or all pairs simultaneously during a number of consecutive cardiac cycles) to be able to detect and locate a myocardial infarction. That is, during each impedance measuring session, a number of impedance measurments from the different electrode pairs are obtained. For example, a measurement including four electrode pairs will provide four impedance values.

Furthermore, myocardial infarction detection means 27' comprises a myocardial infarct detector 35 adapted to evaluate the measured impedances by detecting changes in the impedances that is consistent with a myocardial infarction and to determine a location of the myocardial infarction using the evaluation. In one embodiment, the myocardial infarct detector 35 is adapted to compare the measured impedances with a reference impedance template stored in a template memory 36 to detect an occurrence of a myocardial infarction and a location of the myocardial infarction from the result of the comparison. Alternatively, the reference impedance template may be stored in the storage means 25. Moreover, the reference template can be obtained and created before the parameter monitoring session is initiated, e.g. the impedance measurement session, and updated periodically. In one embodiment, the impedance measurements sessions are synchronized with the heartbeats of the patients, for example, at the end of diastole.

The myocardial infarct detector 35 may be adapted to determine impedance value ratios for each cardiac cycle by determining a maximum impedance and a minimum impedance for each electrode pair during the cardiac cycle. By comparison with the template, it is possible to identify whether a myocardial infarction has occurred. For example, an impedance value ratio being below an impedance value ratio threshold is determined to be consistent with a myocardial infarction. Further, by comparing the impedance value ratios being below the threshold, a location of the myocardial infarction can be determined. In this embodiment, the impedance value ratio being smallest is determined to indicate the location of the myocardial infarction. That is, the electrode pair providing the impedance measurement curve having the smallest difference between the maximum impedance value and the minimum impedance value during a cardiac cycle is determined to be the electrode pair being closest to the detected myocardial infarction and, hence, the location of the myocardial can be determined. The myocardial infarct detector 35 is adapted to send an instruction or message to the controller 24 informing the controller 24 that a myocardial infarction has been detected and the location of the myocardial infarction, i.e. as defined by the electrode pair being determined to be closest to the detected myocardial infarction.

In another embodiment of the present invention, the myocardial infarct detector 35 is adapted to calculate a maximum time derivative of each measured impedance curve, i.e. for each electrode pair. By comparing the calculated maximum time derivates with the template, it is possible to identify whether a myocardial infarction has occurred. For example, a maximum impedance time derivative being below a predetermined impedance time derivative threshold is determined to be consistent with a myocardial infarction. Further, in this embodiment, the maximum impedance time derivative being the lowest of the maximum impedance time derivatives being below the impedance time derivative threshold is determined to indicate the location of the myocardial infarction. That is, the electrode pair providing the impedance measurement curve having the lowest maximum impedance time derivative is determined to be the electrode pair being closest to the detected myocardial infarction. The myocardial infarct detector 35 is adapted to send an instruction or message to the controller 24 informing the controller 24 that a myocardial infarction has been detected and the location of the myocardial infarction, i.e. as defined by the electrode pair being determined to be closest to the detected myocardial infarction.

As the person skilled within the art realizes, there are a number of other conceivable variations or alternatives to the embodiments described above. For example, the morphology of the obtained impedance curves may be compared with an impedance template to determine the occurrence and location of a myocardial infarction. In one embodiment, the part of the impedance curve at systole, i.e. after the QRS-complex, is studied and compared with a reference curve obtained with the same electrode configuration at normal conditions, i.e. at conditions when no myocardial infarction is present.

Moreover, the myocardial infarct detector may be adapted to, after an initiation of a therapy session, monitor impedances obtained by at least the electrode pair that indicated the location of the myocardial infarction to determine whether the obtained impedances indicate that the therapy session should be terminated and/or whether treatment parameters should be adjusted. The therapy parameters can be adjusted during the treatment procedure. For example, a higher light intensity can be used during an initial therapy period and the light intensity can be reduced during a second period after the initial period. Alternatively, a constant light intensity but an adjusted intermittence can be utilized, e.g. the periods of light delivery can be adjusted or shorter intervals between the periods of light delivery are used.

In one embodiment, the myocardial infarct detector is adapted to determine impedance value ratios for successive cardiac cycles and to determine that the therapy session should be terminated if a predetermined number of the impedance value ratios are found to be above a predetermined impedance value ratio threshold. Alternatively, the therapy parameters can be adjusted, for example, shorter intervals between the periods of light delivery can be used if a predetermined number of the impedance value ratios are found to be below a predetermined impedance value ratio threshold.

In a further embodiment, the myocardial infarct detector is adapted to calculate maximum time derivatives of the measured impedance curves for successive cardiac cycles and to determine that the therapy session should be terminated if a predetermined number of the maximum impedance time derivatives are found to be above a predetermined impedance time derivative threshold. Alternatively, the therapy parameters can be adjusted, for example, shorter intervals between the periods of light delivery can be used if a predetermined number of the impedance value ratios are found to be below a predetermined impedance value ratio threshold.

Turning now to Fig. 3b, an embodiment of the myocardial infarction detection means adapted to determine an occurrence of a myocardial infarction and to determine the location of the myocardial infarction using electrical activity of the heart of the patient impedances will be described. The myocardial infarction detection means 27" comprises means for sensing electrical activity 43 of the heart including an intracardiac electrogram measuring circuit connected to electrodes incorporated in the medical leads 22a and/or 22b, which will be described in more detail below with reference to Fig. 4. In one embodiment, each medical lead comprises a plurality of ring electrodes arranged along the respective lead and a tip electrode and the means for sensing electrical activity 43 may be connected to the electrodes via a switching device 44. The switching device 44 may be arranged in the implantable medical device 20 and is adapted to switch between selected electrode(-s) of the medical lead (-s) 22a, 22b to obtain signals indicative of the electrical activity of the heart from different electrode(-s) and/or combination of electrodes. A person skilled within the art may design such a switching device based on the switching device described in U.S. 5,423,873. Thereby, the electrical activity of the heart can be measured using different electrodes and/or combination of electrodes, for example, at a first ring electrode of the first medical lead 22a and a second ring electrode of the first medical lead 22a, at a first ring electrode of the first medical lead 22a and at a first ring electrode of the second medical lead 22b, and/or at a first ring electrode of the second medical lead 22b and at a second ring electrode of the second medical lead 22b. The means for sensing electrical activity 43 is adapted to perform sensing sessions according to a myocardial infarction monitoring protocol, wherein the electrical activity at different sensors, electrodes and/or combinations of electrodes are measured according to a predetermined sequence (e.g. one electrode after another during consecutive cardiac cycles or a number of electrodes simultaneously during a number of consecutive cardiac cycles) to be able to sense electrical activity and obtain intracardiac electrograms for different electrodes and/or combinations of electrodes.

Furthermore, the myocardial infarction detection means 27" includes a myocardial infarct detector 45 adapted to evaluate the obtained intracardiac electrograms to detect changes being consistent with a myocardial infarction and to determine a location of the myocardial infarction using the evaluation. In one embodiment, the myocardial infarct detector is adapted to determine a ST segment elevation of each obtained intracardiac electrogram and compare them with a reference template stored in a template memory 46 to detect an occurrence of a myocardial infarction and a location of the myocardial infarction from the result of the comparison. Alternatively, the reference impedance template may be stored in the storage means 25. Moreover, the reference template can be obtained and created before the parameter monitoring session is initiated, e.g. the impedance measurement session, and updated periodically.

In this embodiment, it is determined whether the ST segment elevation is above a predetermined ST segment threshold and in such a case; it is determined to be consistent with the occurrence of a myocardial infarction. The intracardiac electrogram having the largest ST segment elevation of the ST segments being above the predetermined ST segment threshold is determined to indicate the location of the myocardial infarction. That is, the electrode and/or electrode combination providing the intracardiac electrogram curve having the largest ST segment elevation during a cardiac cycle is determined to be the electrode and/or electrode combination being closest to the detected myocardial infarction and, hence, the location of the myocardial can be determined. The myocardial infarct detector 45 is adapted to send an instruction or message to the controller 24 informing the controller 24 that a myocardial infarction has been detected and the location of the myocardial infarction, i.e. as defined by the electrode and/or electrode combination being determined to be closest to the detected myocardial infarction. In one embodiment, the amplitude of a cardiac signal is measured during a short interval after the detection of R-wave. For example, the interval is about 40 - 150 ms after the R-wave detection. Measured amplitude is compared with a predetermined reference amplitude value and when the measured amplitude exceeds the reference value, a myocardial infarction is indicated.

Moreover, the myocardial infarct detector may be adapted to, after an initiation of a therapy session, monitor intracardiac electrograms obtained by at least an electrode and/or an electrode combination indicating the location of the myocardial infarction to determine whether obtained intracardiac electrograms indicate that the therapy session should be terminated and/or the treatment parameters should be adjusted. For example, a higher light intensity can be used during an initial therapy period and the light intensity can be reduced during a second period after the initial period. Alternatively, a constant light intensity but an adjusted intermittence can be utilized, e.g. the periods of light delivery can be adjusted or shorter intervals between the periods of light delivery are used.

The myocardial infarct detector may be adapted to determine ST segments for successive cardiac cycles after the initiation of the therapy session and to determine that therapy session should be terminated if a predetermined number of the obtained ST segment elevations are found to be below a predetermined ST segment elevation threshold. Alternatively, the therapy parameters can be adjusted, for example, shorter intervals between the periods of light delivery can be used if a predetermined number of the impedance value ratios are found to be above a predetermined impedance value ratio threshold.

According to a further embodiment of the present invention, the myocardial infarction detection means 27 comprises circuitry for detecting the occurrence and location of a myocardial infarction using both impedances and intracardiac electrogram. In this case, the occurrence and location of a myocardial infarction can be detected by using impedances and the healing process can be monitored by means of intracardiac electrograms, for example, by studying the ST elevation.

With reference to Fig. 4, an embodiment of a medical lead in accordance with the present invention will be described. The medical lead 50 comprises a number of tines 51 for fixating the lead 50 at the cardiac tissue. An annular tip electrode 52 is arranged at the tip of the lead and will, after the implantation, abut against the cardiac tissue. A light emitting diode 54 is arranged at the centre of the tip portion of the lead. Further, an array of ring electrodes 55a-55c are arranged along an outer periphery 56 of the medical lead. An array of light emitting diodes 57a-57d is arranged along the outer periphery 56.

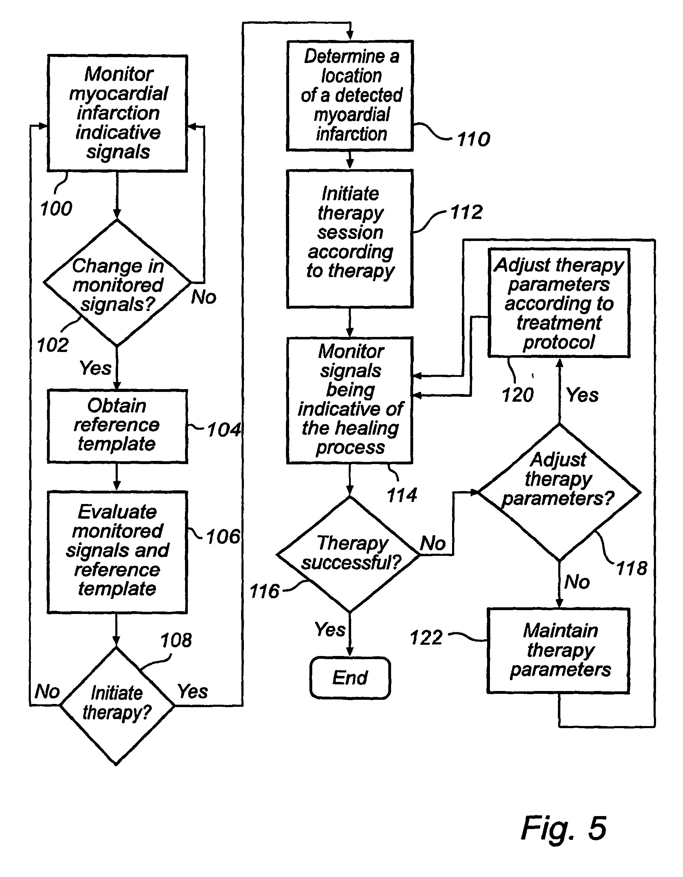

Turning now to Fig. 5, a high-level flow chart of an embodiment of the method for treating a myocardial infarction of a heart of a patient with therapeutic light using an implantable medical device according to the present invention is shown. At step 100, signals indicative of a myocardial infarction is monitored, for example, impedances of cardiac tissue or electrical activity for determining intracardiac electrograms as discussed above. This monitoring, i.e. the measuring or sensing sessions can be initiated at periodic intervals or can be performed continuously. At step 102, a determination is constantly or at regular intervals made in the myocardial infarct detector 35, 45 as to whether there has been any change in the signal being monitored of sufficient magnitude to indicate the possibility of an occurrence of a myocardial infarction. If no change, or if the magnitude of the change is too small, the algorithm returns to step 100. According to an embodiment, the algorithm waits for a predetermined period of time before it returns to step 100.

On the other hand, if a change that indicates the occurrence of a myocardial infarction is detected, the algorithm proceeds to step 104 where a reference template is obtained. The reference template may be a predetermined template stored in the template memory 36, 46, in the memory of the implantable medical device 20, or a template obtained and created by using measurements performed during a period when no myocardial indicative change in the monitored signals is detected. This created template may be updated periodically. Then, at step 106, the obtained data, e.g. the morphology of the impedance curves, a maximum impedance time derivative for the different impedance curves, or a ST elevation of the different intracardiac electrograms, are compared with the reference template. At step 108, it is checked whether the comparison indicates a deviation such that an occurrence of a myocardial infarction can be established and, thus, whether a delivery of therapy is justified. If the comparison indicates that the deviation is not sufficient to justify an initiation of a therapy, the algorithm returns to step 100.

If the deviation indicates that therapy should be initiated, the algorithm proceeds to step 110, where a location of the established myocardial detection is determined by using the obtained data, for example, the impedance curves or the intracardiac electrograms as described above. For example, the ST elevation being the largest or the minimum difference between the maximum impedance value and the minimum impedance value indicate which electrode and/or electrode combination that is closest to the detected myocardial infarction. Then, at step 112, a therapy session is initiated in accordance with a therapy protocol, which may include predetermined or adjustable treatment parameters such as emitting intervals of the therapeutic light, intensity of the emitted therapeutic light, wavelength of the emitted light, or intermittence of the emitted therapeutic light. The therapy protocol may be stored in the memory 25 of the implantable medical device 20 or in the memory of the myocardial detection means 27', 27".

At step 114, signals being indicative of the healing process are continuously monitored after the initiation of the therapy session. As described above, impedance signals and/or intracardiac electrograms may be used for this determination. At step 116, it is determined whether the therapy should be terminated based on the therapy protocol. If yes, the therapy is ended. On the other hand if no, the algorithm proceeds to step 118, where it is checked whether the therapy parameters should be adjusted. For example, shorter intervals between the periods of light delivery can be used if a predetermined number of the impedance value ratios, i.e. for a number of successive cardiac cycles, are found to be within a predetermined impedance value ratio interval or if the ST elevation, i.e. for a number of successive cardiac cycles, is found to be within a predetermined ST elevation value interval. If yes, the algorithm proceeds to step 120, where the therapy parameters are adjusted in accordance with the therapy protocol. Then, the algorithm returns to step 114, where the therapy is continued with the new adjusted parameters. Alternatively, the algorithm may proceed to step 112, where a new therapy session is initiated with the new adjusted parameters. On the other hand, if it is determined that the therapy parameters should not be adjusted at step 118, the algorithm proceeds to step 122 where the therapy parameters are maintained. Thereafter, the algorithm returns to step 114, where the therapy is continued with the maintained therapy parameters. Alternatively, the algorithm may proceed to step 112, where a new therapy session is initiated with the maintained parameters.

The present invention applies to implantable medical devices such as implantable pacemakers including biventricular pacemakers, pacemakers capable of delivering pacing to the atrium, the ventricle, or both the atrium and the ventricle (i.e. left ventricle and/or right ventricle), as well as devices, which are capable of delivering one or more cardioversion or defibrillation shocks.

In a further embodiment of the present invention, the control circuit 24 is adapted to, at detection of an occurrence of a myocardial infarction, send a notification to a medical care institution, e.g. a hospital or a care centre, via a communication unit of the medical device 10, 20, 30 and at least one external radio communication network such as wireless LAN ("Local Area Network"), GSM ("Global System for Mobile communications"), or UMTS ("Universal Mobile Telecommunications System"). For a given communication method, a multitude of standard and/or proprietary communication protocols may be used. For example, and without limitation, wireless (e.g. radio frequency pulse coding, spread spectrum frequency hopping, time-hopping, etc.) and other communication protocols (e.g. SMTP, FTP, TCP/IP) may be used. Other proprietary methods and protocols may also be used. The notification may include at least the patient identity, the occurrence of a myocardial infarction and/or the location of the detected infarct within the heart. The communication unit may be adapted to communicate with an extracorporeal communication device, e.g. mobile phone, a pager or a PDA ("Personal Digital Assistant"), which is adapted to receive the notification and to transmit it via said communication network further to the medical care institution. Alternatively, the communication unit may be adapted to communicate with a home monitoring unit located in the home of the patient. The home monitoring unit is adapted to communicate with the care institution via a telephone link. Furthermore, the notification may include a geographical location of the patient, for example, by means of a GPS ("Global Positioning System") unit arranged in the communication device. Thereby, it is possible for the care institution to obtain an early notification of the infarct of a patient and, additionally, the position of the patient and hence the patient can be given care at an early stage of an infarction.

In a further embodiment, not part of the present invention, an extracorporeal therapy unit may be connected to a medical lead according to the present invention for supplying, for example, power to the light emitting means or, in case of light conducting optical fibres in the medical lead for supplying therapeutic light. Furthermore, an extracorporeal therapy unit comprising a lead in form of a guide wire including light emitting means in accordance with the present invention may be used to treat the detected infarct since the medical personnel controlling the therapy unit may be provided with the location of the detected infarct via the implanted medical device.

Although an exemplary embodiment of the present invention has been shown and described, it will be apparent to those having ordinary skill in the art that a number of changes, modifications, or alterations to the inventions as described herein may be made. Thus, it is to be understood that the above description of the invention and the accompanying drawings is to be regarded as a nonlimiting example thereof and that the scope of protection is defined by the appended patent claims.

## Claims

1. An implantable medical device (10; 20; 30) including a pulse generator (21) adapted to produce cardiac stimulating pacing pulses and being connected to at least one medical lead (12, 14; 22a, 22b; 50) for delivering said pulses to cardiac tissue of a heart (16) of a patient, said implantable medical device comprising:
myocardial infarction detection means (27; 27'; 27") adapted to detect a myocardial infarction and to identify a location of said myocardial infarction;
a therapy circuit (23) connected to a plurality of light emitting means (32a, 32b; 54, 57a, 57b, 57c, 57d) arranged in said at least one medical lead (12, 14; 22a, 22b; 50) adapted to emit therapeutic light; and
a control circuit (24) connected to said myocardial infarction detection means (27; 27'; 27") and to said therapy circuit (23), said control circuit (24) being adapted to initiate a therapy session via said therapy circuit (23), wherein one or more of said plurality of light emitting means (32a, 32b; 54, 57a, 57b, 57c, 57d) are selectively activated to emit said therapeutic light towards a detected location of a myocardial infarction at detection of an occurrence of a myocardial infarction.

2. The implantable medical device according to claim 1, wherein said therapy circuit (23) is adapted to activate said light emitting means (32a, 32b; 54, 57a, 57b, 57c, 57d) to emit said therapeutic light according to a treatment protocol.

3. The implantable medical device according to claim 2, wherein said treatment protocol includes treatment parameters comprising: emitting intervals of said therapeutic light, intensity of said emitted therapeutic light, wavelength of said emitted light, and intermittence of said emitted therapeutic light.

4. The implantable medical device according to any one of claims 1-3, wherein each of said plurality of light emitting means (54, 57a, 57b, 57c, 57d) comprises at least one light emitting diode (54, 57a, 57b, 57c, 57d).

5. The implantable medical device according to claims 1-4, wherein said light emitting means (54, 57a, 57b, 57c, 57d) are arranged in an array arranged along an outer surface of a lead body of said medical lead (12, 14 ; 22a, 22b; 50).

6. The implantable medical device according to any one of claims 1-3, wherein each of said plurality of light emitting means (32a, 32b) comprises at least one optical fibre (32a, 32b) adapted to conduct light emitted from at least one light source (31) arranged in said implantable medical device (30), wherein said therapy circuit (23) is adapted to selectively activate said at least one light source (31) and/or at least one optical fibre (32a, 32b) such that light conducted in one or more optical fibres (32a, 32b) emanates from said one or more optical fibres (32a, 32b) towards said detected location.

7. The implantable medical device according to claim 6, wherein the at least one light source (31) is a laser source.

8. The implantable medical device according to any one of preceding claims, wherein said medical lead (50) comprises a plurality of electrodes (52, 55a, 55b, 55c), said myocardial infarction detection means (27') comprising
an impedance measuring circuit (33) connected to said electrodes (52, 55a, 55b, 55c) being adapted to:
apply excitation current pulses between respective electrode pairs including at least a first and at least a second electrode; and
measure the impedance in the tissues between said at least first and said at least second electrode of said electrode pairs to the excitation current pulses; and
a myocardial infarct detector (35) adapted to evaluate said measured impedances by detecting changes in said impedances being consistent with a myocardial infarction and to determine a location of said myocardial infarction using said evaluation.

9. The implantable medical device according to claim 8, wherein said myocardial infarct detector (35) is adapted to compare measured impedances with a stored reference impedance template to detect an occurrence of a myocardial infarction and a location of said myocardial infarction from the result of the comparison.

10. The implantable medical device according to claim 9, wherein said myocardial infarct detector (35) is adapted to
determine impedance value ratios for a cardiac cycle by determining a maximum impedance and a minimum impedance, respectively, measured by the impedance measuring circuit during a cardiac cycle;
determine an impedance value ratio being below a predetermined impedance value ratio threshold to be consistent with a myocardial infarction; and
determine the impedance value ratio being smallest of the impedance value ratios being below said predetermined impedance value ratio threshold to indicate the location of the myocardial infarction.

11. The implantable medical device according to claim 9 or 10, wherein said myocardial infarct detector (35) is adapted to
calculate a respective maximum time derivative of the measured impedance ;
determine a maximum impedance time derivative being below a predetermined impedance time derivative threshold to be consistent with a myocardial infarction; and
determine the maximum impedance time derivative being lowest of the maximum impedance time derivatives being below said predetermined impedance time derivative threshold to indicate the location of the myocardial infarction.

12. The implantable medical device according to any one of preceding claims, wherein said medical lead (50) comprises a plurality of electrodes (52, 55a, 55b, 55c), said myocardial infarction detection means (27") comprising
an intracardiac electrogram measuring circuit (43) connected to said electrodes (52, 55a, 55b, 55c) and being adapted to measure intracardiac electrograms using respective electrode pairs; and
a myocardial infarct detector (45) adapted to evaluate said intracardiac electrograms to detect changes being consistent with a myocardial infarction and to determine a location of said myocardial infarction using said evaluation.

13. The implantable medical device according to claims 8-12, wherein said myocardial infarct detector (35) is, after an initiation of a therapy session, adapted to
monitor impedances obtained by at least an electrode pair indicating the location of said myocardial infarction to determine whether said impedances indicate that said therapy session should be terminated and/or said treatment parameters should be adjusted.

14. The implantable medical device according to claim 13, wherein said myocardial infarct detector (35) is adapted to
determine impedance value ratios for successive cardiac cycles; and
determine that said therapy session should be terminated if a predetermined number of said impedance value ratios are found to be above said impedance value ratio threshold.

15. The implantable medical device according to claim 13, wherein said myocardial infarct detector (35) is adapted to
calculate maximum time derivatives of the measured impedance for successive cardiac cycles; and
determine that said therapy session should be terminated if a predetermined number of said maximum impedance time derivatives are found to be above a predetermined impedance time derivative threshold.

16. The implantable medical device according to any one of preceding claims 12-15, wherein said myocardial infarct detector (45) is adapted to
monitor intracardiac electrograms obtained by at least an electrode pair indicating the location of said myocardial infarction to determine whether said intracardiac electrograms indicate that said therapy session should be terminated and/or said treatment parameters should be adjusted.

17. The implantable medical device according to claim 16, wherein said myocardial infarct detector (45) is adapted to
determine ST segments for successive cardiac cycles; and
determine that said therapy session should be terminated if a predetermined number of said ST segment elevations are found to be below a predetermined ST segment elevation threshold.

18. The implantable medical device according to any one of preceding claims, wherein said light emitting means (31, 32a, 32b; 54, 57a, 57b, 57c, 57d) is adapted to emit light having a wavelength in the range of 600 nm - 1000 nm.

19. The implantable medical device according to any one of preceding claims, wherein said control circuit (24) is adapted to, at detection of an occurrence of a myocardial infarction, send a notification to a medical care institution via a communication unit of said medical device (10; 20; 30) and at least one external communication network, said notification including at least an identity of the patient and information related to a detected myocardial infarction.

20. The implantable medical device according to claim R19, wherein said communication unit of said medical device (10; 20; 30) is adapted to communicate with an extracorporeal communication device, said communication device being adapted to receive said notification and to transmit said notification via said communication network to said medical care institution.

21. The implantable medical device according to claim 19, wherein said communication unit of said medical device (10; 20; 30) is adapted to communicate with an extracorporeal home monitoring unit connected to said at least one communication network, said home monitoring unit being adapted to receive said notification and to transmit said notification via said communication network to said medical care institution.

22. The implantable medical device according to any one of preceding claims, further comprising a notifying device adapted to, at detection of an occurrence of a myocardial infarction, notify said patient of the event that a myocardial infarct has been detected and/or that therapy has been initiated.

23. A medical lead (12, 14; 22a, 22b; 50) connected to an implantable medical device (10; 20 ; 30) including a pulse generator (21) adapted to produce cardiac stimulating pacing pulses, said medical lead comprising:
a plurality of electrodes (52, 55a, 55b, 55c) connected to myocardial infarction detection means (27; 27'; 27") arranged in said implantable medical device (10; 20 ; 30), wherein said myocardial infarction detection means (27; 27'; 27") is adapted to detect a myocardial infarction and to identify a location of said myocardial infarction;
a plurality of light emitting means (31, 32a, 32b; 54, 57a, 57b, 57c, 57d) adapted to emit therapeutic light, said light emitting means (31, 32a, 32b; 54, 57a, 57b, 57c, 57d) being connected to a therapy circuit (23) and to a control circuit (24) arranged in said implantable medical device (10; 20 ;30), wherein said control circuit (24) is adapted to initiate a therapy session via said therapy circuit (23), wherein one or more of said plurality of light emitting means (31, 32a, 32b; 54, 57a, 57b, 57c, 57d) are selectively activated to emit said therapeutic light towards a detected location of a myocardial infarction at detection of an occurrence of a myocardial infarction.

24. The medical lead according to claim 23, wherein each of said plurality of light emitting means (54, 57a, 57b, 57c, 57d) comprises at least one light emitting diode.

25. The medical lead according to claim 23 or 24; wherein said light emitting means (54, 57a, 57b, 57c, 57d) are arranged in an array arranged along an outer surface,of a lead body of said medical lead (12, 14; 22a, 22b; 50).

26. The medical lead according to claim 23-25 wherein each of said plurality of light emitting means (31, 32a, 32b) comprises at least one optical fibre adapted to conduct light emitted from at least one light source (31) arranged in said implantable medical device (30), wherein said therapy circuit (23) is adapted to selectively activate said at least one light source (31) and/or at least one optical fibre such that light conducted in one or more optical fibres emanates from said one or more optical fibres towards said detected location.

27. The medical lead according to any one of preceding claims 23-25, wherein said light emitting means (31, 32a, 32b; 54, 57a, 57b, 57c, 57d) is adapted to emit coherent and monochromatic light.

## Patentansprüche

1. Implantierbare medizinische Vorrichtung (10; 20; 30), die einen Impulsgenerator (21) aufweist, der angepasst ist, Herzstimulationserregungsimpulse zu erzeugen, und mit mindestens einer medizinischen Leitung (12, 14; 22a, 22b; 50) zum Abgeben der Impulse an Herzgewebe eines Herzes (16) eines Patienten verbunden ist, wobei die implantierbare medizinische Vorrichtung Folgendes umfasst:
Mittel (27; 27'; 27") zum Nachweis eines Myokardinfarkts, die angepasst sind, einen Myokardinfarkt nachzuweisen und den Ort des Myokardinfarkts festzustellen,
eine Behandlungsschaltung (23), die mit mehreren lichtaussendenden Mitteln (32a, 32b; 54, 57a, 57b, 57c, 57d) verbunden ist, die in der mindestens einen medizinischen Leitung (12, 14; 22a, 22b; 50) angeordnet und angepasst sind, therapeutisches Licht auszusenden, und
eine Steuerschaltung (24), die mit den Mitteln (27; 27'; 27") zum Nachweis eines Myokardinfarkts und mit der Behandlungsschaltung (23) verbunden ist, wobei die Steuerschaltung (24) angepasst ist, eine Behandlungssitzung über die Behandlungsschaltung (23) zu beginnen, wobei ein oder mehrere der mehreren lichtaussendenden Mittel (32a, 32b; 54, 57a, 57b, 57c, 57d) gezielt aktiviert werden, um das therapeutische Licht in Richtung eines erfassten Orts eines Myokardinfarkts beim Nachweis des Auftretens eines Myokardinfarkts auszusenden.

2. Implantierbare medizinische Vorrichtung nach Anspruch 1, wobei die Behandlungsschaltung (23) angepasst ist, die lichtaussendenden Mittel (32a, 32b; 54, 57a, 57b, 57c, 57d) zu aktivieren, damit sie therapeutisches Licht entsprechend einem Behandlungsprotokoll aussenden.

3. Implantierbare medizinische Vorrichtung nach Anspruch 2, wobei das Behandlungsprotokoll Behandlungsparameter aufweist, die Folgendes umfassen:
Aussendeintervalle des therapeutischen Lichts, Intensität des ausgesendeten therapeutischen Lichts, Wellenlänge des ausgesendeten Lichts und Unterbrechungen des ausgesendeten therapeutischen Lichts.

4. Implantierbare medizinische Vorrichtung nach einem der Ansprüche 1 bis 3, wobei jedes der mehreren lichtaussendenden Mittel (54, 57a, 57b, 57c, 57d) mindestens eine Leuchtdiode (54, 57a, 57b, 57c, 57d) umfasst.

5. Implantierbare medizinische Vorrichtung nach einem der Ansprüche 1 bis 4, wobei die lichtaussendenden Mittel (54, 57a, 57b, 57c, 57d) in einer Anordnung angeordnet sind, die entlang einer Außenfläche eines Leitungskörpers der medizinischen Leitung (12, 14; 22a, 22b; 50) angeordnet ist.

6. Implantierbare medizinische Vorrichtung nach einem der Ansprüche 1 bis 3, wobei jedes der mehreren lichtaussendenden Mittel (32a, 32b) mindestens einen Lichtwellenleiter (32a, 32b) umfasst, der angepasst ist, Licht zu leiten, das von mindestens einer Lichtquelle (31) ausgesendet wird, die in der implantierbaren medizinischen Vorrichtung (30) angeordnet ist, wobei die Behandlungsschaltung (23) angepasst ist, gezielt die mindestens eine Lichtquelle (31) und/oder mindestens einen Lichtwellenleiter (32a, 32b) derart zu aktivieren, dass Licht, das in einem oder mehreren Lichtwellenleitern (32a, 32b) geleitet wird, aus dem einem oder den mehreren Lichtwellenleiter(n) (32a, 32b) in Richtung des erfassten Orts ausstrahlt.

7. Implantierbare medizinische Vorrichtung nach Anspruch 6, wobei die mindestens eine Lichtquelle (31) eine Laserquelle ist.

8. Implantierbare medizinische Vorrichtung nach einem der vorhergehenden Ansprüche, wobei die medizinische Leitung (50) mehrere Elektroden (52, 55a, 55b, 55c) umfasst, wobei die Mittel (27') zum Nachweis des Myokardinfarkts Folgendes umfassen:
eine Impedanzmessschaltung (33), die mit den Elektroden (52, 55a, 55b, 55c) verbunden und angepasst ist:
Erregungsstromimpulse zwischen jeweiligen Elektrodenpaaren, die mindestens eine erste und mindestens eine zweite Elektrode aufweisen, abzugeben, und
die Impedanz in den Geweben zwischen der mindestens ersten und der mindestens zweiten Elektrode der Elektrodenpaare auf die Erregungsstromimpulse zu messen, und
einen Myokardinfarktdetektor (35), der angepasst ist, die gemessenen Impedanzen durch Erfassen von Veränderungen der Impedanzen, die mit einem Myokardinfarkt übereinstimmen, zu beurteilen und einen Ort des Myokardinfarkts unter Verwendung der Beurteilung zu bestimmen.

9. Implantierbare medizinische Vorrichtung nach Anspruch 8, wobei der Myokardinfarktdetektor (35) angepasst ist, gemessene Impedanzen mit einem gespeicherten Bezugsimpedanzmuster zu vergleichen, um aus dem Ergebnis des Vergleichs das Auftreten eines Myokardinfarkts nachzuweisen und einen Ort des Myokardinfarkts zu erfassen.

10. Implantierbare medizinische Vorrichtung nach Anspruch 9, wobei der Myokardinfarktdetektor (35) angepasst ist:
Impedanzwertverhältnisse für einen Herzzyklus durch Bestimmen einer maximalen Impedanz beziehungsweise einer minimalen Impedanz zu bestimmen, die von der Impedanzmessschaltung während eines Herzzyklus gemessen wird,
festzustellen, dass ein Impedanzwertverhältnis, das unter einer festgelegten Impedanzwertverhältnisschwelle liegt, mit einem Myokardinfarkt übereinstimmt, und
das Impedanzwertverhältnis zu ermitteln, das das geringste der Impedanzwertverhältnisse ist, die unter der festgelegten Impedanzwertverhältnisschwelle liegen, um den Ort des Myokardinfarkts anzugeben.

11. Implantierbare medizinische Vorrichtung nach Anspruch 9 oder 10, wobei der Myokardinfarktdetektor (35) angepasst ist:
eine entsprechende maximale zeitliche Ableitung der gemessenen Impedanz zu berechnen,
festzustellen, dass eine maximale zeitliche Ableitung der Impedanz, die unter einer festgelegten Schwelle der zeitlichen Ableitung der Impedanz liegt, mit einem Myokardinfarkt übereinstimmt, und
die maximale zeitliche Ableitung der Impedanz zu ermitteln, die die geringste der maximalen zeitlichen Ableitungen der Impedanz ist, die unter der festgelegten Schwelle der zeitlichen Ableitung der Impedanz liegen, um den Ort des Myokardinfarkts anzugeben.

12. Implantierbare medizinische Vorrichtung nach einem der vorhergehenden Ansprüche, wobei die medizinische Leitung (50) mehrere Elektroden (52, 55a, 55b, 55c) umfasst, wobei die Mittel (27") zum Nachweis des Myokardinfarkts Folgendes umfassen:
eine intrakardiale Elektrogrammmessschaltung (43), die mit den Elektroden (52, 55a, 55b, 55c) verbunden und angepasst ist, unter Verwendung jeweiliger Elektrodenpaare intrakardiale Elektrogramme zu bestimmen, und
einen Myokardinfarktdetektor (45), der angepasst ist, die intrakardialen Elektrogramme zu beurteilen, um Veränderungen, die mit einem Myokardinfarkt übereinstimmen, zu erfassen und einen Ort des Myokardinfarkts unter Verwendung der Beurteilung zu bestimmen.

13. Implantierbare medizinische Vorrichtung nach Anspruch 8 bis 12, wobei der Myokardinfarktdetektor (35), nach Beginn einer Behandlungssitzung, angepasst ist:
Impedanzen, die von mindestens einem Elektrodenpaar erhalten werden, das auf den Ort des Myokardinfarkts hinweist, zu überwachen, um zu bestimmen, ob die Impedanzen darauf hinweisen, dass die Behandlungssitzung beendet werden sollte und/oder die Behandlungsparameter angepasst werden sollten.

14. Implantierbare medizinische Vorrichtung nach Anspruch 13, wobei der Myokardinfarktdetektor (35) angepasst ist:
Impedanzwertverhältnisse für aufeinanderfolgende Herzzyklen zu bestimmen, und
zu bestimmen, dass die Behandlungssitzung beendet werden sollte, wenn festgestellt wird, dass eine festgelegte Zahl der Impedanzwertverhältnisse über der Impedanzwertverhältnisschwelle liegt.

15. Implantierbare medizinische Vorrichtung nach Anspruch 13, wobei der Myokardinfarktdetektor (35) angepasst ist:
maximale zeitliche Ableitungen der gemessenen Impedanz für aufeinanderfolgende Herzzyklen zu berechnen, und
zu ermitteln, dass die Behandlungssitzung beendet werden sollte, wenn festgestellt wird, dass eine festgelegte Zahl der maximalen zeitlichen Ableitungen der Impedanz über einer festgelegten Schwelle der zeitlichen Ableitung der Impedanz liegt.

16. Implantierbare medizinische Vorrichtung nach einem der vorhergehenden Ansprüche 12 bis 15, wobei der Myokardinfarktdetektor (45) angepasst ist:
intrakardiale Elektrogramme, die von mindestens einem Elektrodenpaar erhalten werden, das auf den Ort des Myokardinfarkts hinweist, zu überwachen, um zu bestimmen, ob die intrakardialen Elektrogramme darauf hinweisen, dass die Behandlungssitzung beendet werden sollte und/oder die Behandlungsparameter angepasst werden sollten.

17. Implantierbare medizinische Vorrichtung nach Anspruch 16, wobei der Myokardinfarktdetektor (45) angepasst ist:
ST-Strecken für aufeinanderfolgende Herzzyklen zu bestimmen, und
zu ermitteln, dass die Behandlungssitzung beendet werden sollte, wenn festgestellt wird, dass eine festgelegte Zahl der ST-Streckenhebungen unter einer festgelegten ST-Streckenhebungsschwelle liegt.

18. Implantierbare medizinische Vorrichtung nach einem der vorhergehenden Ansprüche, wobei das lichtaussendende Mittel (31, 32a, 32b, 54, 57a, 57b, 57c, 57d) angepasst ist, Licht mit einer Wellenlänge im Bereich von 600 nm bis 1000 nm auszusenden.

19. Implantierbare medizinische Vorrichtung nach einem der vorhergehenden Ansprüche, wobei die Steuerschaltung (24) angepasst ist, beim Nachweis des Auftretens eines Myokardinfarkts eine Mitteilung über eine Kommunikationseinheit der medizinischen Vorrichtung (10; 20; 30) und mindestens ein externes Kommunikationsnetz an eine medizinische Einrichtung zu senden, wobei die Mitteilung mindestens eine Kennung des Patienten und Informationen enthält, die sich auf einen nachgewiesenen Myokardinfarkt beziehen.

20. Implantierbare medizinische Vorrichtung nach Anspruch 19, wobei die Kommunikationseinheit der medizinischen Vorrichtung (10; 20; 30) angepasst ist, mit einer Kommunikationsvorrichtung außerhalb des Körpers zu kommunizieren, wobei die Kommunikationsvorrichtung angepasst ist, die Mitteilung zu empfangen und die Mitteilung über das Kommunikationsnetz an die medizinische Einrichtung zu übertragen.

21. Implantierbare medizinische Vorrichtung nach Anspruch 19, wobei die Kommunikationseinheit der medizinischen Vorrichtung (10; 20; 30) angepasst ist, mit einer Fernüberwachungseinheit außerhalb des Körpers zu kommunizieren, die mit dem mindestens einen Kommunikationsnetz verbunden ist, wobei die Fernüberwachungseinheit angepasst ist, die Mitteilung zu empfangen und die Mitteilung über das Kommunikationsnetz an die medizinische Einrichtung zu übertragen.

22. Implantierbare medizinische Vorrichtung nach einem der vorhergehenden Ansprüche, ferner umfassend eine Mitteilungsvorrichtung, die angepasst ist, den Patienten beim Nachweis des Auftretens eines Myokardinfarkts über den Umstand zu informieren, dass ein Myokardinfarkt nachgewiesen wurde und/oder dass mit der Behandlung begonnen wurde.

23. Medizinische Leitung (12, 14; 22a, 22b; 50), die mit einer implantierbaren medizinischen Vorrichtung (10; 20; 30) verbunden ist, die einen Impulsgenerator (21) aufweist, der angepasst ist, Herzstimulationserregungsimpulse zu erzeugen, wobei die medizinische Leitung Folgendes umfasst:
mehrere Elektroden (52, 55a, 55b, 55c), die mit Mitteln (27; 27'; 27") zum Nachweis eines Myokardinfarkts verbunden sind, die in der implantierbaren medizinischen Vorrichtung (10; 20 ; 30) angeordnet sind, wobei die Mittel (27; 27'; 27") zum Nachweis eines Myokardinfarkts angepasst sind, einen Myokardinfarkt nachzuweisen und einen Ort des Myokardinfarkts festzustellen,
mehrere lichtaussendende Mittel (31, 32a, 32b; 54, 57a, 57b, 57c, 57d), die angepasst sind, therapeutisches Licht auszusenden, wobei die lichtaussendenden Mittel (31, 32a, 32b; 54, 57a, 57b, 57c, 57d) mit einer Behandlungsschaltung (23) und mit einer Steuerschaltung (24) verbunden sind, die in der implantierbaren medizinischen Vorrichtung (10; 20; 30) angeordnet sind, wobei die Steuerschaltung (24) angepasst ist, eine Behandlungssitzung über die Behandlungsschaltung (23) zu beginnen, wobei ein oder mehrere der mehreren lichtaussendenden Mittel (31, 32a, 32b; 54, 57a, 57b, 57c, 57d) gezielt aktiviert werden, um das therapeutische Licht in Richtung eines erfassten Orts eines Myokardinfarkts beim Nachweis des Auftretens eines Myokardinfarkts auszusenden.

24. Medizinische Leitung nach Anspruch 23, wobei jedes der mehreren lichtaussendenden Mittel (54, 57a, 57b, 57c, 57d) mindestens eine Leuchtdiode umfasst.

25. Medizinische Leitung nach Anspruch 23 oder 24, wobei die lichtaussendenden Mittel (54, 57a, 57b, 57c, 57d) in einer Anordnung angeordnet sind, die entlang einer Außenfläche eines Leitungskörpers der medizinischen Leitung (12, 14; 22a, 22b; 50) angeordnet ist.

26. Medizinische Leitung nach Anspruch 23 bis 25, wobei jedes der mehreren lichtaussendenden Mittel (31, 32a, 32b) mindestens einen Lichtwellenleiter umfasst, der angepasst ist, Licht zu leiten, das von mindestens einer Lichtquelle (31) ausgesendet wird, die in der implantierbaren medizinischen Vorrichtung (30) angeordnet ist, wobei die Behandlungsschaltung (23) angepasst ist, gezielt die mindestens eine Lichtquelle (31) und/oder mindestens einen Lichtwellenleiter derart zu aktivieren, dass Licht, das in einem oder mehreren Lichtwellenleitern geleitet wird, aus dem einem oder den mehreren Lichtwellenleitern in Richtung des erfassten Orts ausstrahlt.

27. Medizinische Leitung nach einem der vorhergehenden Ansprüche 23 bis 26, wobei das lichtaussendende Mittel (31, 32a, 32b; 54, 57a, 57b, 57c, 57d) angepasst ist, kohärentes und monochromatisches Licht auszusenden.

## Revendications

1. Dispositif médical implantable (10 ; 20 ; 30) comprenant un générateur d'impulsions (21) adapté pour produire des impulsions excitatrices de stimulation cardiaque et étant connecté à au moins un conducteur médical (12, 14 ; 22a, 22b ; 50) pour fournir lesdites impulsions au tissu cardiaque d'un coeur (16) d'un patient, ledit dispositif médical implantable comprenant :
des moyens de détection d'infarctus du myocarde (27 ; 27' ; 27") adaptés pour détecter un infarctus du myocarde et pour identifier une localisation dudit infarctus du myocarde ;
un circuit de thérapie (23) connecté à une pluralité de moyens émetteurs de lumière (32a, 32b ; 54, 57a, 57b, 57c, 57d) agencés dans ledit au moins un conducteur médical (12, 14 ; 22a, 22b ; 50) adaptés pour émettre de la lumière thérapeutique ; et
un circuit de commande (24) connecté auxdits moyens de détection d'infarctus du myocarde (27 ; 27' ; 27") et audit circuit de thérapie (23), ledit circuit de commande (24) étant adapté pour lancer une session de thérapie via ledit circuit de thérapie (23), dans lequel un ou plusieurs de ladite pluralité de moyens émetteurs de lumière (32a, 32b ; 54, 57a, 57b, 57c, 57d) sont activés de manière sélective pour émettre ladite lumière thérapeutique vers une localisation détectée d'un infarctus du myocarde lors de la détection d'une présence d'un infarctus du myocarde.

2. Dispositif médical implantable selon la revendication 1, dans lequel ledit circuit de thérapie (23) est adapté pour activer lesdits moyens émetteurs de lumière (32a, 32b ; 54, 57a, 57b, 57c, 57d) pour émettre ladite lumière thérapeutique selon un protocole de traitement.

3. Dispositif médical implantable selon la revendication 2, dans lequel ledit protocole de traitement comprend des paramètres de traitement comprenant : intervalles d'émission de ladite lumière thérapeutique, intensité de ladite lumière thérapeutique émise, longueur d'onde de ladite lumière émise, et intermittence de ladite lumière thérapeutique émise.

4. Dispositif médical implantable selon l'une quelconque des revendications 1 à 3, dans lequel chacun de ladite pluralité de moyens émetteurs de lumière (54, 57a, 57b, 57c, 57d) comprend au moins une diode électroluminescente (54, 57a, 57b, 57c, 57d).

5. Dispositif médical implantable selon l'une quelconque des revendications 1 à 4, dans lequel lesdits moyens émetteurs de lumière (54, 57a, 57b, 57c, 57d) sont agencés dans une disposition agencée le long d'une surface extérieure d'un corps de conducteur dudit conducteur médical (12, 14 ; 22a, 22b ; 50).

6. Dispositif médical implantable selon l'une quelconque des revendications 1 à 3, dans lequel chacun de ladite pluralité de moyens émetteurs de lumière (32a, 32b) comprend au moins une fibre optique (32a, 32b) adaptée pour conduire de la lumière émise par au moins une source lumineuse (31) agencée dans ledit dispositif médical implantable (30), dans lequel ledit circuit de thérapie (23) est adapté pour activer de manière sélective ladite au moins une source lumineuse (31) et/ou au moins une fibre optique (32a, 32b) de sorte que de la lumière conduite dans une ou plusieurs fibres optiques (32a, 32b) émane desdites une ou plusieurs fibres optiques (32a, 32b) vers ladite localisation détectée.

7. Dispositif médical implantable selon la revendication 6, dans lequel la au moins une source lumineuse (31) est une source laser.

8. Dispositif médical implantable selon l'une quelconque des revendications précédentes, dans lequel ledit conducteur médical (50) comprend une pluralité d'électrodes (52, 55a, 55b, 55c), lesdits moyens de détection d'infarctus du myocarde (27') comprenant
un circuit de mesure d'impédance (33) connecté auxdites électrodes (52, 55a, 55b, 55c) qui est adapté pour :
appliquer des impulsions de courant d'excitation entre des paires respectives d'électrodes comprenant au moins une première et au moins une seconde électrode ; et
mesurer l'impédance dans les tissus entre ladite au moins une première et ladite au moins une seconde électrode desdites paires d'électrodes aux impulsions de courant d'excitation ; et
un détecteur d'infarctus du myocarde (35) adapté pour évaluer lesdites impédances mesurées en détectant des changements dans lesdites impédances qui sont concordants avec un infarctus du myocarde et pour déterminer une localisation dudit infarctus du myocarde en utilisant ladite évaluation.

9. Dispositif médical implantable selon la revendication 8, dans lequel ledit détecteur d'infarctus du myocarde (35) est adapté pour comparer des impédances mesurées à un modèle d'impédance de référence stocké pour détecter une présence d'un infarctus du myocarde et une localisation dudit infarctus du myocarde à partir du résultat de la comparaison.

10. Dispositif médical implantable selon la revendication 9, dans lequel ledit détecteur d'infarctus du myocarde (35) est adapté pour
déterminer des rapports de valeurs d'impédance pour un cycle cardiaque en déterminant respectivement une impédance maximale et une impédance minimale, mesurées par le circuit de mesure d'impédance pendant un cycle cardiaque ;
déterminer qu'un rapport de valeurs d'impédance qui est inférieur à un seuil prédéterminé de rapport de valeurs d'impédance est concordant avec un infarctus du myocarde ; et
déterminer le rapport de valeurs d'impédance étant le plus faible des rapports de valeurs d'impédance qui sont inférieurs audit seuil prédéterminé de rapport de valeurs d'impédance pour indiquer la localisation de l'infarctus du myocarde.

11. Dispositif médical implantable selon la revendication 9 ou 10, dans lequel ledit détecteur d'infarctus du myocarde (35) est adapté pour
calculer une dérivée temporelle maximale respective de l'impédance mesurée ;
déterminer qu'une dérivée temporelle d'impédance maximale qui est inférieure à un seuil prédéterminé de dérivée temporelle d'impédance est concordante avec un infarctus du myocarde ; et
déterminer la dérivée temporelle d'impédance maximale étant la plus faible des dérivées temporelles d'impédance maximale qui sont inférieures audit seuil prédéterminé de dérivée temporelle d'impédance pour indiquer la localisation de l'infarctus du myocarde.

12. Dispositif médical implantable selon l'une quelconque des revendications précédentes, dans lequel ledit conducteur médical (50) comprend une pluralité d'électrodes (52, 55a, 55b, 55c), lesdits moyens de détection d'infarctus du myocarde (27") comprenant
un circuit de mesure d'électrogramme endocavitaire (43) connecté auxdites électrodes (52, 55a, 55b, 55c) et étant adapté pour mesurer des électrogrammes endocavitaires en utilisant des paires d'électrodes respectives ; et
un détecteur d'infarctus du myocarde (45) adapté pour évaluer lesdits électrogrammes endocavitaires pour détecter des changements qui sont concordants avec un infarctus du myocarde et pour déterminer une localisation dudit infarctus du myocarde en utilisant ladite évaluation.

13. Dispositif médical implantable selon les revendications 8 à 12, dans lequel ledit détecteur d'infarctus du myocarde (35) est, après un lancement de session de thérapie, adapté pour
surveiller des impédances obtenues par au moins une paire d'électrodes indiquant la localisation dudit infarctus du myocarde pour déterminer si lesdites impédances indiquent que ladite session de thérapie devrait être terminée et/ou si lesdits paramètres de traitement devraient être ajustés.

14. Dispositif médical implantable selon la revendication 13, dans lequel ledit détecteur d'infarctus du myocarde (35) est adapté pour
déterminer des rapports de valeurs d'impédance pour des cycles cardiaques successifs ; et
déterminer que ladite session de thérapie devrait être terminée si un nombre prédéterminé desdits rapports de valeurs d'impédance est détecté comme étant au-dessus dudit seuil de rapport de valeurs d'impédance.

15. Dispositif médical implantable selon la revendication 13, dans lequel ledit détecteur d'infarctus du myocarde (35) est adapté pour
calculer des dérivées temporelles maximales de l'impédance mesurée pour des cycles cardiaques successifs ; et
déterminer que ladite session de thérapie devrait être terminée si un nombre prédéterminé desdites dérivées temporelles d'impédance maximales est détecté comme étant au-dessus d'un seuil prédéterminé de dérivée temporelle d'impédance.

16. Dispositif médical implantable selon l'une quelconque des revendications précédentes 12 à 15, dans lequel ledit détecteur d'infarctus du myocarde (45) est adapté pour
surveiller des électrogrammes endocavitaires obtenus par au moins une paire d'électrodes indiquant la localisation dudit infarctus du myocarde pour déterminer si lesdits électrogrammes endocavitaires indiquent que ladite session de thérapie devrait être terminée et/ou si lesdits paramètres de traitement devraient être ajustés.

17. Dispositif médical implantable selon la revendication 16, dans lequel ledit détecteur d'infarctus du myocarde (45) est adapté pour
déterminer des segments ST pour des cycles cardiaques successifs ; et
déterminer que ladite session de thérapie devrait être terminée si un nombre prédéterminé desdites élévations de segment ST est détecté comme étant inférieur à un seuil prédéterminé d'élévation de segment ST.

18. Dispositif médical implantable selon l'une quelconque des revendications précédentes, dans lequel ledit moyen émetteur de lumière (31, 32a, 32b ; 54, 57a, 57b, 57c, 57d) est adapté pour émettre de la lumière présentant une longueur d'onde située dans la plage allant de 600 nm à 1000 nm.

19. Dispositif médical implantable selon l'une quelconque des revendications précédentes, dans lequel ledit circuit de commande (24) est adapté pour, lors de la détection d'une présence d'un infarctus du myocarde, envoyer une notification à un établissement de soins médicaux via une unité de communication dudit dispositif médical (10 ; 20 ; 30) et au moins un réseau de communication extérieur, ladite notification comprenant au moins une identité du patient et des informations relatives à un infarctus du myocarde détecté.

20. Dispositif médical implantable selon la revendication 19, dans lequel ladite unité de communication dudit dispositif médical (10 ; 20 ; 30) est adaptée pour communiquer avec un dispositif de communication extracorporel, ledit dispositif de communication étant adapté pour recevoir ladite notification et pour transmettre ladite notification via ledit réseau de communication audit établissement de soins médicaux.

21. Dispositif médical implantable selon la revendication 19, dans lequel ladite unité de communication dudit dispositif médical (10 ; 20 ; 30) est adaptée pour communiquer avec une unité de surveillance à domicile extracorporelle connectée audit au moins un réseau de communication, ladite unité de surveillance à domicile étant adaptée pour recevoir ladite notification et pour transmettre ladite notification via ledit réseau de communication audit établissement de soins médicaux.

22. Dispositif médical implantable selon l'une quelconque des revendications précédentes, comprenant en outre un dispositif de notification adapté pour, lors de la détection d'une présence d'un infarctus du myocarde, notifier ledit patient de l'événement qu'un infarctus du myocarde a été détecté et/ou qu'une thérapie a été lancée.

23. Conducteur médical (12, 14 ; 22a, 22b ; 50) connecté à un dispositif médical implantable (10 ; 20 ; 30) comprenant un générateur d'impulsions (21) adapté pour produire des impulsions excitatrices de stimulation cardiaque, ledit conducteur médical comprenant :
une pluralité d'électrodes (52, 55a, 55b, 55c) connectées à des moyens de détection d'infarctus du myocarde (27 ; 27' ; 27") agencés dans ledit dispositif médical implantable (10 ; 20 ; 30), dans lequel lesdits moyens de détection d'infarctus du myocarde (27, 27' ; 27") sont adaptés pour détecter un infarctus du myocarde et pour identifier une localisation dudit infarctus du myocarde ;
une pluralité de moyens émetteurs de lumière (31, 32a, 32b ; 54, 57a, 57b, 57c, 57d) adaptés pour émettre de la lumière thérapeutique, lesdits moyens émetteurs de lumière (31, 32a, 32b ; 54, 57a, 57b, 57c, 57d) étant connectés à un circuit de thérapie (23) et à un circuit de commande (24) agencés dans ledit dispositif médical implantable (10 ; 20 ; 30), dans lequel ledit circuit de commande (24) est adapté pour lancer une session de thérapie via ledit circuit de thérapie (23), dans lequel un ou plusieurs de ladite pluralité de moyens émetteurs de lumière (31, 32a, 32b ; 54, 57a, 57b, 57c, 57d) sont activés de manière sélective pour émettre ladite lumière thérapeutique vers une localisation détectée d'un infarctus du myocarde lors de la détection d'une présence d'un infarctus du myocarde.

24. Conducteur médical selon la revendication 23, dans lequel chacun de ladite pluralité de moyens émetteurs de lumière (54, 57a, 57b, 57c, 57d) comprend au moins une diode électroluminescente.

25. Conducteur médical selon la revendication 23 ou 24, dans lequel lesdits moyens émetteurs de lumière (54, 57a, 57b, 57c, 57d) sont agencés dans une disposition agencée le long d'une surface extérieure d'un corps de conducteur dudit conducteur médical (12, 14 ; 22a, 22b ; 50).

26. Conducteur médical selon les revendications 23 à 25, dans lequel chacun de ladite pluralité de moyens émetteurs de lumière (31, 32a, 32b) comprend au moins une fibre optique adaptée pour conduire de la lumière émise par au moins une source lumineuse (31 ) agencée dans ledit dispositif médical implantable (30), dans lequel ledit circuit de thérapie (23) est adapté pour activer de manière sélective ladite au moins une source lumineuse (31) et/ou au moins une fibre optique de sorte que de la lumière conduite dans une ou plusieurs fibres optiques émane desdites une ou plusieurs fibres optiques vers ladite localisation détectée.

27. Conducteur médical selon l'une quelconque des revendications précédentes 23 à 26, dans lequel ledit moyen émetteur de lumière (31, 32a, 32b ; 54, 57a, 57b, 57c, 57d) est adapté pour émettre une lumière cohérente et monochromatique.
